# EUROPEAN PATENT SPECIFICATION

(11) **EP 3 234 226 B1**
(45) Date of publication and mention of the grant of the patent: **10.04.2019**
(21) Application number: 15813393.4
(22) Date of filing: 17.12.2015
(51) Int. Cl.: C25B 3/04, C25B 11/03, C25B 11/04

(54) **METHOD FOR MANUFACTURING 2,3-BUTANEDIOL**
VERFAHREN ZUR HERSTELLUNG VON 2,3-BUTANDIOL
PROCÉDÉ POUR PRODUIRE DU 2,3-BUTANEDIOL

(30) Priority: 18.12.2014 EP 14198812
(43) Date of publication of application: 25.10.2017
(73) Proprietor: Fundación Tecnalia Research & Innovation, 20009 Donostia - San Sebastian (ES)
(72) Inventor: OCHOA GÓMEZ, José Ramón, 01510 Miñano (ES); GARCÍA LUIS, Alberto, 20009 Donostia (San Sebastián) (ES); FERNÁNDEZ CARRETERO, Francisco José, 20009 Donostia (San Sebastián) (ES); LORENZO IBARRETA, Leire, 01510 Miñano (ES); PRIETO FERNÁNDEZ, Soraya, 01510 Miñano (ES)
(74) Representative: ZBM Patents - Zea, Barlocci & Markvardsen
(86) International application number: PCT/EP2015/080187
(87) International publication number: WO 2016/097122

(56) References cited:
- WO-A1-2013/134220

## Description

The present disclosure relates to methods for manufacturing 2,3-butanediol.

### BACKGROUND

2,3-Butanediol (2,3-BDO) is a chemical with important current and potential industrial applications, e.g. as antifreeze, as raw material for methyl ethyl ketone and 1,3-butadiene manufacturing by dehydration, and even as liquid fuel due to its heating value of 27198 kJ.kg⁻¹ which is comparable to those of methanol (22081 kJ.kg⁻¹) and ethanol (29055 kJ.kg⁻¹). Other potential applications include the manufacture of printing inks, perfumes, fumigants, moistening and softening agents, explosives and plasticizers, and as a carrier for pharmaceuticals.

Almost the totality of the 2,3-BDO manufacturing processes described are based on fermentation of carbohydrates using many bacterial species as shown by both academic and patent literature. All these methods have in common as main drawbacks a very low 2,3-BDO productivity, usually ranging from 1 to 3 g/L/h, and a low 2,3-BDO titer in the final culture broth, usually below 120 g/L, and much more usually below 100 g/L. The latter fact, together with the highly complex chemical composition of the culture broth, lead to cumbersome methods for isolation and purification of 2,3-BDO with the corresponding economic penalties.

There are also some chemical routes for obtaining 2,3-BDO. Thus, in CN 103193596A 2,3-BDO is synthesized from a mixture of an alcohol (e.g., methanol, ethanol, propanol and butanol) and mixed C4 hydrocarbons by oxidation with hydrogen peroxide in the presence of titanium silicalite modified with aluminium oxide as catalyst. However, this process leads to a low 2,3-BDO selectivity of 41%. In JPH0441447 2,3-BDO is produced by means of photocatalysis by irradiating ethanol with light resulting from a high-intensity ultraviolet laser in the presence of hydrogen peroxide, process which is not industrially feasible.

WO2013134220 relates to a process for the electrocatalytic hydrogenation and/or hydrodeoxygenation of biomass-derived bio-oil components and to the related electrode.

Recently, the present inventors have applied for patent (WO2016012634) wherein a method for obtaining 2,3-BDO by hydrogenation of 3-hydroxybutanone in an aqueous medium using heterogeneous catalysts based on nickel and noble metals is disclosed. In this process, 2,3-BDO is obtained in yields as high as 98% at hydrogen pressures higher than 20 bar, preferably 40 bar, and temperatures above 75 °C. Although results reported in such an Application are very good and leads to an industrially feasible procedure for 2,3-BDO manufacturing, a process not using hydrogen and able to operate at room temperature would be much more desirable due to both economic and safety reasons.

The only document in scientific literature related to 3-hydroxybutanone electroreduction is that of M.M. Baizer et al. (Electrochemical conversion of 2,3-butanediol to 2-butanone in undivided flow cells: a paired synthesis, Journal of Applied Electrochemistry 14 (1984) 197-208). In this paper, a procedure was developed for converting 2,3-butanediol in *ca.* 10% aqueous solution to 2-butanone (methyl ethyl ketone) by passing it through a porous anode at which it is selectively oxidized to 3-hydroxybutanone by electrogenerated NaBrO and then pumping to a porous cathode at which 3-hydroxybutanone is reduced to 2-butanone. Therefore, in this paper 3-hydroxybutanone electroreduction yields 2-butanone (methyl ethyl ketone).

### SUMMARY

The present disclosure relates to processes for the preparation of 2,3-butanediol by electroreduction of 3-hydroxybutanone in aqueous media using a cathode comprising a cathodic electrocatalytic material comprising metals from the I B, II B and VIII B Groups of the Periodic Table; their oxides; or mixtures thereof; wherein the process comprises the following steps:
a) forming a solution by mixing 3-hydroxybutanone with an aqueous medium and a supporting electrolyte in such a medium,
b) electrolyzing said solution continuously or discontinuously in an electrochemical reactor by applying a voltage between an anode and the cathode using a direct current (DC) power supply;
wherein the cathode comprises the cathodic electrocatalytic material in the form of a porous metal material or alternatively the cathode comprises the cathodic electrocatalytic material deposited on a porous electrically conductive support; and
wherein the pH of the solution in which the electroreduction of 3-hydroxybutanone is carried out is comprised between 3-4.

### DETAILED DESCRIPTION OF THE INVENTION

3-hydroxybutanone has an asymmetric carbon and consequently it is a chiral molecule. Any one of the stereoisomers as well as their mixtures can be used as a raw material in the process of the present invention. Accordingly, throughout the present invention the term 3-hydroxybutanone encompasses its enantiomers as well as mixtures thereof in any proportions, e.g. a racemic mixture.

As used in the present invention, the term "cathodic electrocatalytic material" refers to the electrocatalytic material of the cathode, which comprises one or more metals from the I B, II B and VIII B Groups of the Periodic Table; their oxides; or mixtures thereof.

As used in the present invention, the terms "cell", "electrochemical cell" and "electrochemical reactor" are interchangeable.

As used in the present invention, aqueous medium means water or a mixture of water with a fully or partially water-miscible solvent, in which the water concentration in the aqueous medium is comprised between 50 and 100 wt%, preferably between 70 and 100 wt%, more preferably between 85 and 100 wt%, and most preferably 100 wt%. Suitable fully or partially water-miscible solvents are those which are not electroactive under the electrolysis conditions of the present invention. Examples of said solvents, but not limited to, are alcohols such as methanol, ethanol, propanol and isopropanol; ethers such as tetrahydrofuran and dioxane; and nitriles such as acetonitrile.

The electrochemical reactor used in the process of the present invention can be any one of those known by a person skilled in the art, e.g. either a tank-type or a flow-through filter press-type electrochemical reactor, with the latter being the preferred one. The electrochemical reactor can be divided or undivided, with this last configuration being the most preferred because leads to both lower power consumption and a lower capital investment. If a divided electrochemical reactor is used, anode and cathode are separated by a material preventing mixing of anolyte (the 3-hydroxybutanone-free solution being fed through the anodic compartment) and catholyte (the 3-hydroxybutanone-containing solution being fed through the cathode compartment) while allows the flow of ions transporting electricity in solution. A cation exchange membrane is the most preferred separating material for divided electrochemical reactors. Examples of cation exchange membranes, but are not limited to, are any one of those marketed under the trademark of Nafion® such as, e.g., Nafion® N-324 and Nafion® N-424.

As anodic materials (anode), DSA (dimensionally stable anodes) are the preferred ones in the method of the present invention. Non limitative examples of DSA anodes are platinum supported on titanium (Pt/Ti) and iridium oxides supported on titanium. PbO₂/Ti can also be used in divided cells.

The cathodic electrocatalytic material used in the method of the present invention is a material comprising metals from the I B, II B and VIII B Groups of the Periodic Table, their oxides, or mixtures thereof; preferably Fe, Co, Ni, Cu, Zn, Ru, Rh, Pd, Ag, Cd, Os, Ir, Pt, their oxides or mixtures thereof; with Ni, Pd, Pt, Ru, Rh and Ir, and mixtures thereof, being more preferred. Also, Nickel alloys can be used as cathode materials. Monel type Ni-Cu alloys are, for instance, a non-limiting illustrative example of a nickel alloy useful in the method of the present invention.

As disclosed herein above, cathode materials are porous. They can be in the form of commercially available such as perforated metal foils, metal felts, metal meshes, metal foams. As a non-limiting example given exclusively for illustrative purposes, if nickel is used as a cathode material, it is preferentially used in a porous form such as, e.g., a nickel mesh with an open area between 55% and 85%, or nickel foams with a porosity of 95% and number of pores/cm ranging between about 6.5 and about 25.

Also as disclosed herein above, alternatively, said metals from the I B, II B and VIII B Groups of the Periodic Table are deposited on a porous electrically conductive support. Non-limiting examples of porous electrically conductive supports are commercially available GDL (gas diffusion layers), both carbon paper type and carbon cloth type, such as, for instance but not limited to, those marketed under the trademarks of Sigracet®, Freudenberg, Spectracarb™, Avcarb® and Toray for GDL carbon paper type, and ELAT™ for GDL carbon cloth type. The coating of the metals on the GDL supports can be made by any one of the known techniques in the art such as by Physical Vapor Deposition (PVD), Chemical Vapour Deposition (CVD), Thermal Spraying, Ink Spraying or electrodeposition. Typical PVD coating processes are evaporation, using cathodic arcs or electron beam sources, and sputtering, using magnetic enhanced sources or "magnetrons", cylindrical or hollow cathode sources. All PVD processes are carried out in vacuum at working pressures typically ranged between 10⁻² to 10⁻⁴ mbar, and generally involve bombardment of the substrate to be coated with energetic positively charged ions. Additionally, reactive gases such as nitrogen, acetylene or oxygen may be introduced into the vacuum chamber during metal deposition to create various coating compositions. The result is a very strong bond between the coating and the support and tailored physical, structural and tribological properties of the film. By way of example, a Pt coating on a Sigracet® GDL-24BC can be done as follows: the GDL sample is introduced in a PVD chamber that comprises a planar magnetron with a Pt cathode and a rotating holder. After a degasing process including heating in vacuum, argon is introduced into the chamber up to a working pressure from 0.16 to 0.6 Pa. A supply of DC current from 200 to 600 W (typically 400 W) to the magnetron initiates the argon plasma ignition and the sputtering of Pt ions from the target, leading to the deposition of a thin Pt film (of the order of few nanometers) on the GDL substrate. Process time ranges from 30 seconds to 2 minutes. In a typical spraying coating process a catalyst deposited on carbon (e.g. Pt), with a catalyst mass respect to carbon between 20 wt% and 40 wt%, is suspended in an appropriate solvent (e.g. iso-propyl alcohol) achieving a concentration between 1 and 5 wt% and more preferably between 2 wt% and 4 wt%. A certain amount of ionomer (e.g. Nafion solution 5 wt%) is added to the mixture, comprised between 5% and 25% of the catalyst mass and most preferably between 10 wt% and 15 wt% of the catalyst mass. The suspension is homogenized combining magnetic stirring with ultra-sonication at room temperature until homogenous ink is achieved. A uniform active layer is formed on a GDL by spraying the catalyst ink on a hot plate at between 70°C and 80°C.

The metal surface density after coating the support is preferably comprised between 10 µg/cm² and 1500 µg/cm², more preferably between 20 µg/cm² and 1000 µg/cm², and most preferably between 30 µg/cm² and 750 µg/cm².

The electroreduction of 3-hydroxybutanone according to the present invention is carried out at a current density comprised between 100 and 10000 A/m², preferably between 250 and 5000 A/m², and more preferably between 500 and 3000 A/m².

The electroreduction of 3-hydroxybutanone according to the present invention is performed in the presence of a supporting electrolyte added to adjust the conductivity of the electrolysis solution and/or to control the selectivity of the reaction. The electrolyte concentration is generally adjusted to a level from about 0.1 to about 20 wt%, preferably from about 1 to about 15 wt%, and more preferably from about 5 to about 10 wt%, based on the total mass of the reaction mixture. Examples of supporting electrolytes in undivided cells and for catholyte when divided cells are used include, but are not limited to, ammonium and alkaline and alkaline earth metals salts of inorganic acids such as sulfuric, phosphoric and nitric acids.

If the process of the present invention is carried out in an divided cell, additional supporting electrolytes for catholyte are ammonium and alkaline and alkaline-earth metals salts of hydrochloric acid, hydrobromic acid and hydrofluoric acid; and supporting electrolytes for anolyte include, but are not limited to, inorganic acids, such as sulfuric and phosphoric acids, as well as ammonium and alkaline and alkaline earth metals salts of said inorganic acids.

As disclosed herein above, pH of electrolyte in undivided cells or pH of catholyte in divided cells is between 3 and 4. pH adjustment can be done by adding a suitable acid, such as, e.g., phosphoric and sulfuric acids, or base, such as, e.g., sodium or potassium hydroxides.

According to the present invention, 3-hydroxybutanone concentration in the solution to be electrolyzed is at least 10 g/L, preferably at least 25 g/L, more preferably at least 50 g/L and the most preferably at least 100 g/L, based on the total volume of solution to be electrolyzed.

The amount of electricity circulated for electroreducing 3-hydroxybutanone to 2,3-BDO according to the method of the present invention is preferably comprised between 50% and 150% of the theoretical one for obtaining a 100% conversion of 3-hydroxybutanone assuming a current efficiency of 100% (2 faradays per mol of 3-hydroxybutanone), more preferably between 75% and 125%, and most preferably between 90% and 125%.

According to the present invention, 3-hydroxybutanone electroreduction to 2,3-BDO is carried out at ambient pressure and at a temperature ranging between room temperature and 10 °C below the boiling point of the aqueous medium, preferably at room temperature.

The method of the present invention can be carried out using either one electrochemical reactor or at least two electrochemical reactors connected in series in such a way that the reaction mixture resulting from the first reactor feeds the second one and so on. If two or more electrochemical reactors connected in series are used both current density and circulated electrical charge decrease from the first electrochemical reactor to the last one. For instance, if two electrochemical reactors connected in series are used, the current density used in the first electrochemical reactors is higher than that used in the second electrochemical reactor; and the fraction of the circulated electrical charge in the first electrochemical reactor, relative to the total charge circulated through both electrochemical reactors, is higher in the first electrochemical reactor than that in the second electrochemical reactor. In this way, electricity is more efficiently employed in electroreducing 3-hydroxybutanone to 2,3-BDO.

The method of the present invention is illustrated below by reference to the examples and examples comparatives which are intended to be only illustrative and are not construed to limit the present invention in any way.

### EXAMPLES

### Example 1

A solution (60 mL) of 3-hydroxybutanone (112.2 g/L) and KH₂PO₄ (5 wt%) in water was recirculated by means of a magnetic pump through the cathode compartment of a divided filter press cell consisting of a Iridium oxide-based DSA anode (20 cm²), a Nafion® N-324 cation exchange membrane separating anode and cathode compartments, and a Pt/Ti plate cathode (20 cm²). Inter-electrode gap was 2 cm. An aqueous 10 wt% sulfuric acid solution was recirculated through the anode compartment by means of another magnetic pump. An electrical current was circulated (2 A, 1000 A/m²) by applying a voltage between anode and cathode using a DC Power Supply. Electrolysis was kept at room temperature (20-25°C) for 2.05 h (100% of the theoretical charge for full conversion of 3-hydroxybutanone assuming a current efficiency of 100%). Initial catholyte pH was 3.78 and final pH 3.68 (mean pH 3.73). After electrolysis completion, the catholyte solution (62 mL) contained a 3-hydroxybutanone concentration of 79.9 g/L and a 2,3-BDO concentration of 10.7 g/L, as shown by HPLC. Therefore, 3-hydroxybutanone conversion was 26.4% (26.4% current yield) and 2,3-BDO yield was 9.6% resulting in a 2,3-BDO selectivity, the ratio between yield and conversion, of 36.3%.

### Example 2

A solution (60 mL) of 3-hydroxybutanone (112.2 g/L) and KH₂PO₄ (5 wt%) in water was recirculated by means of a magnetic pump through an undivided filter press cell consisting of a Iridium oxide-based DSA anode (20 cm²) and a Pt/Ti plate cathode (20 cm²) separated 0.8 cm each other by means of a polypropylene (PP) separator. An electrical current was circulated (3 A, 1500 A/m²) by applying a voltage between anode and cathode using a DC Power Supply. Electrolysis was kept at room temperature (20-25°C) for 1.36 h (100% of the theoretical charge for full conversion of 3-hydroxybutanone assuming a current efficiency of 100%). Initial solution pH was 3.78 and final pH 3.46 (mean pH 3.63). After electrolysis completion, the electrolyzed solution (56.8 mL) contained a 3-hydroxybutanone concentration of 85.5 g/L and a 2,3-BDO concentration of 19.6 g/L, as shown by HPLC. Therefore, 3-hydroxybutanone conversion was 27.9% (27.9% current yield) and 2,3-BDO yield was 16.2% resulting in a 2,3-BDO selectivity of 58.1%.

### Example 3

Electrolysis was carried out as in example 2, but using a Ni plate cathode and a current density of 1000 A/m². Initial solution pH was 3.78 and final pH 3.50 (mean pH 3.64). Electrolysis time was 2.05 h (100% of the theoretical charge). After electrolysis completion, the final electrolyzed solution (57 mL) contained a 3-hydroxybutanone concentration of 65.1 g/L and a 2,3-BDO concentration of 23.4 g/L, as shown by HPLC. Therefore, 3-hydroxybutanone conversion was 44.9% (44.9% current yield) and 2,3-BDO yield 19.4% resulting in a 2,3-BDO selectivity of 43.2%.

### Example 4

A solution (60 mL) of 3-hydroxybutanone (98.5 g/L), KH₂PO₄ (2.5 wt%) and Na₂SO₄ (4 wt%) in water adjusted to pH 3.6 with phosphoric acid, was recirculated by means of a magnetic pump through an undivided filter press cell consisting of a Iridium oxide-based DSA anode (20 cm²) and a Ni foam (20 cm² geometric area, 1.6 mm thickness, 95% porosity, 20 pores/cm, 0.45 g/cm³ apparent density) cathode separated 0.8 cm each other by means of a PP separator. An electrical current was circulated (2 A, 1000 A/m²) by applying a voltage between anode and cathode using a DC Power Supply. Electrolysis was kept at room temperature (20-25°C) for 1.90 h (105.5% of the theoretical charge for full conversion of 3-hydroxybutanone assuming a current efficiency of 100%). Initial solution pH was 3.6 and final pH 3.4 (mean pH 3.5). After electrolysis completion, the electrolyzed solution (56.5 mL) contained a 3-hydroxybutanone concentration of 1.86 g/L and a 2,3-BDO concentration of 88.8 g/L, as shown by HPLC. Therefore, 3-hydroxybutanone conversion was 98.2% (93.1% current yield) and 2,3-BDO yield 83.0% resulting in a 2,3-BDO selectivity of 84.5%.

### Example 5

Electrolysis was carried out as in example 4, but using a 99.6 g/L concentration of 3-hydroxybutanone and a 20 cm² (geometric area) PtOx/Sigracet® GDL-24BC cathode prepared by PVD with a PtOx surface density (calculated by dividing the weight increase of the GDL support after the PVD process by the geometric area of the GDL-24BC support) of 112 µg/cm². For cell assembly, the cathode was stuck on a stainless steel plate, also acting as electricity collector, with a suitable adhesive for carbon. Electrolysis was kept at room temperature (20-25°C) for 1.90 h (104.4% of the theoretical charge for full conversion of 3-hydroxybutanone assuming a current efficiency of 100%). Both initial and final solution pHs were 3.6. After electrolysis completion, the electrolyzed solution (57 mL) contained a 3-hydroxybutanone concentration of 20.1 g/L and a 2,3-BDO concentration of 75.1 g/L, as shown by HPLC. Therefore, 3-hydroxybutanone conversion was 80.9% (77.1% current yield) and 2,3-BDO yield 70.0% resulting in a 2,3-BDO selectivity of 86.5%.

### Example 6

Electrolysis was carried out as in example 5, but using a 20 cm² (geometric area) PtOx/Sigracet® GDL-24BC cathode prepared by PVD with a PtOx surface density (calculated as in example 5) of 133 µg/cm². After electrolysis completion, the electrolyzed solution (57.5 mL) contained a 3-hydroxybutanone concentration of 28.1 g/L and a 2,3-BDO concentration of 75.8 g/L, as shown by HPLC. Therefore, 3-hydroxybutanone conversion was 72.9% (69.9% current yield) and 2,3-BDO yield 71.3% resulting in a 2,3-BDO selectivity of 97.8%.

### Example 7

Electrolysis was carried out as in example 5, but using a 97 g/L concentration of 3-hydroxybutanone and a 20 cm² (geometric area) Pt/Sigracet® GDL-24BC cathode prepared by PVD with a Pt surface density (calculated as in example 5) of 500 µg/cm². For cell assembly, the cathode was stuck on a stainless steel plate, also acting as electricity collector, with a suitable adhesive for carbon. Electrolysis was kept at room temperature (20-25°C) for 1.90 h (107% of the theoretical charge for full conversion of 3-hydroxybutanone assuming a current efficiency of 100%). Initial solution pH was 3.6 and final pH 3.5. After electrolysis completion, the electrolyzed solution (58 mL) contained a 3-hydroxybutanone concentration of 21.1 g/L and a 2,3-BDO concentration of 72.1 g/L, as shown by HPLC. Therefore, 3-hydroxybutanone conversion was 78.9% (73.8% current yield) and 2,3-BDO yield 70.3% resulting in a 2,3-BDO selectivity of 89.1%.

### Example 8

Electrolysis was carried out as in example 5, but using a 110.6 g/L concentration of 3-hydroxybutanone and a 5 wt% concentration of KH₂PO₄ as well as a 20 cm² (geometric area) Pt₃Co/Sigracet® GDL-24BC cathode prepared by PVD with a Pt₃Co surface density (calculated as in example 5) of 30 µg/cm². Electrolysis time was 1.90 h corresponding to 94% of the theoretical charge for full conversion of 3-hydroxybutanone assuming a current efficiency of 100%. Initial solution pH was 3.8 and final pH 3.7. After electrolysis completion, the electrolyzed solution (55.5 mL) contained a 3-hydroxybutanone concentration of 64.9 g/L and a 2,3-BDO concentration of 35.1 g/L, as shown by HPLC. Therefore, 3-hydroxybutanone conversion was 46.2% (49.1% current yield) and 2,3-BDO yield 29.1% resulting in a 2,3-BDO selectivity of 63.0%.

### Example comparative 1 (not of the invention)

Electrolysis was carried out as in example 5 but using a 101.1 g/L concentration of 3-hydroxybutanone and a 20 cm² (geometric area) Sigracet® GDL-24BC cathode. Electrolysis time was 1.90 h corresponding to 102.8% of the theoretical charge for full conversion of 3-hydroxybutanone assuming a current efficiency of 100%. Initial solution pH was 3.8 and final pH 3.7. After electrolysis completion, the electrolyzed solution (57.8 mL) contained a 3-hydroxybutanone concentration of 25.5 g/L, a 2,3-BDO concentration of 7.3 g/L, and a methyl ethyl ketone concentration of 41.7 g/L, as shown by HPLC. Therefore, 3-hydroxybutanone conversion was 75.7% (73.6% current yield), 2,3-BDO yield was 6.8% (a 2,3-BDO selectivity of 9.0%) and methyl ethyl ketone (MEK) yield was 48.5% (a MEK selectivity of 64%).

This example shows clearly how the use of a porous GDL as a cathode containing no metal yields selectively MEK instead of 2,3-BDO.

## Claims

1. A process for the preparation of 2,3-butanediol by electroreduction of 3-hydroxybutanone in aqueous media using a cathode comprising a cathodic electrocatalytic material comprising metals from the I B, II B and VIII B Groups of the Periodic Table; their oxides; or mixtures thereof, wherein the process comprises the following steps:
a) forming a solution by mixing 3-hydroxybutanone with an aqueous medium and a supporting electrolyte in such a medium,
b) electrolyzing said solution continuously or discontinuously in an electrochemical reactor by applying a voltage between an anode and the cathode using a direct current power supply;
wherein the cathode comprises the cathodic electrocatalytic material in the form of a porous metal material or alternatively the cathode comprises the cathodic electrocatalytic material deposited on a porous electrically conductive support; and
wherein the pH of the solution in which the electroreduction of 3-hydroxybutanone is carried out is comprised between 3-4.

2. The process according to claim 1, wherein the anode is a dimensionally stable anode.

3. The process according to any of the claims 1-2, wherein the anode is selected from the group comprising iridium oxide supported on titanium, platinum supported on titanium (Pt/Ti), and PbO₂/Ti.

4. The process according to any of the claims 1-3, wherein the metals from the I B, II B and VIII B Groups of the Periodic Table are selected from the group consisting of Fe, Co, Ni, Cu, Zn, Ru, Rh, Pd, Ag, Cd, Os, Ir, Pt, and mixtures thereof.

5. The process according to any of the claims 1-4, wherein the metals from the I B, II B and VIII B Groups of the Periodic Table are selected from the group consisting of Ni, Pd, Pt, Ru, Rh and Ir, and mixtures thereof.

6. The process according to any of the claims 1-5, wherein the cathodic electrocatalytic material is selected from nickel alloys.

7. The process according to any of the claims 1-6, wherein the cathode comprises Ni foam or a metal selected from the group consisting of Ni, Pd, Pt, Ru, Rh and Ir, and mixtures thereof deposited on a gas diffusion layer of carbon paper type or carbon cloth type.

8. The process according to claim 7, wherein the cathode is formed by coating the cathodic electrocatalytic material on the porous electrically conductive support and wherein the metal or metal oxide surface density after coating the porous electrically conductive support is comprised between 10 µg/cm² and 1500 µg/cm².

9. The process according to any of claims 1-8, wherein the electroreduction is carried out at a current density comprised between 100 and 10000 A/m².

10. The process according to any of claims 1-9, wherein the electroreduction is carried out at ambient pressure and at a temperature ranging between room temperature and 10 °C below the boiling point of the aqueous medium.

11. The process according to any of claims 1-10, wherein the aqueous medium is water or a mixture of water with a fully or partially water-miscible solvent, in which the water concentration in the aqueous medium is comprised between 50 and 100 wt%, and wherein the fully or partially water-miscible solvent is selected from the group consisting of alcohols, ethers and nitriles.

12. The process according to any of the claims 1-11, wherein the supporting electrolyte concentration in the solution in which the electroreduction of 3-hyroxybutanone is carried out is from 0.1 to 20 wt%, based on the total mass of the solution.

13. The process according to any of the claims 1-12, wherein the supporting electrolyte in the solution in which the electroreduction of 3-hyroxybutanone is carried out is selected from the group of ammonium and alkaline or alkaline earth metals salts of inorganic acids.

14. The process according to any of the claims 1-13, wherein the 3-hydroxybutanone concentration in the solution to be electrolyzed is at least 10 g/L, based on the total volume of solution to be electrolyzed.

15. The process according to any of the claims 1-14, wherein the amount of electricity circulated for electroreducing 3-hydroxybutanone to 2,3-BDO is comprised between 50% and 150% of the theoretical one for obtaining a 100% conversion of 3-hydroxybutanone assuming a current efficiency of 100%.

## Patentansprüche

1. Ein Verfahren zur Herstellung von 2,3-Butandiol durch Elektroreduktion von 3-Hydroxy-2-butanon in einem wässrigen Medium unter Verwendung von einer Kathode umfassend ein kathodisches elektrokatalytisches Material umfassend Metalle aus den Gruppen I B, II B und VIII B des Periodensystems; deren Oxyde; oder Mischungen davon, wobei das Verfahren die folgenden Schritte umfasst:
a) das Bilden von einer Lösung, indem 3-Hydroxy-2-butanon mit einem wässrigen Medium und einem Trägerelektrolyt in einem solchen Medium gemischt wird,
b) das kontinuierliche oder diskontinuierliche Elektrolysieren der Lösung in einem elektrochemischen Reaktor durch Anlegen von einer Spannung zwischen einer Anode und der Kathode unter Verwendung von einer Gleichspannungsstromversorgung;
wobei die Kathode das kathodische elektrokatalytische Material in Form von einem porösen metallischen Material umfasst oder, ersatzweise, die Kathode das kathodische elektrokatalytische Material abgeschieden auf einem porösen elektrisch leitenden Träger umfasst; und
wobei der pH-Wert der Lösung, in der die Elektroreduktion des 3-Hydroxy-2-butanons durchgeführt wird bei zwischen 3-4 liegt.

2. Das Verfahren nach Anspruch 1, wobei die Anode eine formstabile Anode ist.

3. Das Verfahren nach einem der Ansprüche 1-2, wobei die Anode ausgewählt ist aus der Gruppe umfassend auf Titan getragenes Iridiumoxid, auf Titan getragenes Platin (Pt/Ti), und PbO₂/Ti.

4. Das Verfahren nach einem der Ansprüche 1-3, wobei die Metalle aus den Gruppen I B, II B und VIII B des Periodensystems ausgewählt sind aus der Gruppe bestehend aus Fe, Co, Ni, Cu, Zn, Ru, Rh, Pd, Ag, Cd, Os, Ir, Pt, und Mischungen davon.

5. Das Verfahren nach einem der Ansprüche 1-4, wobei die Metalle aus den Gruppen I B, II B und VIII B des Periodensystems ausgewählt sind aus der Gruppe bestehend aus Ni, Pd, Pt, Ru, Rh und Ir, und Mischungen davon.

6. Das Verfahren nach einem der Ansprüche 1-5, wobei das kathodische elektrokatalytische Material aus Nickellegierungen ausgewählt ist.

7. Das Verfahren nach einem der Ansprüche 1-6, wobei die Kathode Ni-Schaum oder ein Metall ausgewählt aus der Gruppe bestehend aus Ni, Pd, Pt, Ru, Rh und Ir, und Mischungen davon abgeschieden auf einer Gasdiffusionsschicht des Kohlepapier- oder Kohlegewebetyps umfasst.

8. Das Verfahren nach Anspruch 7, wobei die Kathode durch Beschichtung des kathodischen elektrokatalytischen Materials auf den porösen elektrisch leitenden Träger gebildet ist und wobei die Dichte des Metalls oder der Metalloxidoberfläche nach der Beschichtung des porösen elektrisch leitenden Trägers bei zwischen 10 µg/cm² und 1.500 µg/cm² liegt.

9. Das Verfahren nach einem der Ansprüche 1-8, wobei die Elektroreduktion bei einer elektrischen Stromdichte von zwischen 100 und 10.000 A/m² durchgeführt wird.

10. Das Verfahren nach einem der Ansprüche 1-9, wobei die Elektroreduktion bei Umgebungsdruck und bei einer Temperatur zwischen der Raumtemperatur und einer Temperatur 10 °C unterhalb des Siedepunkts des wässrigen Mediums durchgeführt wird.

11. Das Verfahren nach einem der Ansprüche 1-10, wobei das wässrige Medium Wasser oder eine Mischung aus Wasser mit einem vollständig oder teilweise wasserlöslichen Lösungsmittel ist, in der die Wasserkonzentration im wässrigen Medium zwischen 50 und 100 Gew.-% beträgt, und wobei das vollständig oder teilweise wasserlösliche Lösungsmittel ausgewählt aus der Gruppe bestehend aus Alkoholen, Ethern und Nitrilen ist.

12. Das Verfahren nach einem der Ansprüche 1-11, wobei die Konzentration des Trägerelektrolyts in der Lösung, in der die Elektroreduktion des 3-Hydroxy-2-butanons durchgeführt wird von 0,1 bis 20 Gew.-%, bezogen auf die gesamte Masse der Lösung, beträgt.

13. Das Verfahren nach einem der Ansprüche 1-12, wobei der Trägerelektrolyt in der Lösung, in der die Elektroreduktion des 3-Hydroxy-2-butanons durchgeführt wird, ausgewählt aus der Gruppe von Ammonium und Salzen von Alkali- oder Erdalkalimetallen von anorganischen Säuren ist.

14. Das Verfahren nach einem der Ansprüche 1-13, wobei die Konzentration des 3-Hydroxy-2-butanons in der zu elektrolysierenden Lösung mindestens 10 g/L, bezogen auf das gesamte Volumen der zu elektrolysierenden Lösung, beträgt.

15. Das Verfahren nach einem der Ansprüche 1-14, wobei die Menge des zur Elektroreduktion des 3-Hydroxy-2-butanons auf 2,3-BDO zu strömenden Stroms zwischen 50% und 150% der zum Erreichen von einer Konversion von 100% des 3-Hydroxy-2-butanons theoretischen Menge, unter Annahme von einer Stromausbeute von 100%, beträgt.

## Revendications

1. Un procédé de préparation de butane-2,3-diol par électroréduction de 3-hydroxybutanone dans un milieu aqueux en utilisant une cathode comprenant un matériau électrocatalytique cathodique comprenant des métaux des groupes I B, II B et VIII B du tableau périodique ; leurs oxydes ; ou des mélanges de ceux-ci, dans lequel le procédé comprend les étapes suivantes :
a) former une solution en mélangeant de la 3-hydroxybutanone avec un milieu aqueux et un électrolyte de support dans ledit milieu,
b) électrolyser ladite solution de manière continue ou discontinue dans un réacteur électrochimique en appliquant une tension entre une anode et la cathode en utilisant une source d'alimentation en courant continu ;
dans lequel la cathode comprend le matériau électrocatalytique cathodique sous forme d'un matériau métallique poreux ou, de façon alternative, la cathode comprend le matériau électrocatalytique cathodique déposé sur un support électriquement conducteur poreux ; et
dans lequel le pH de la solution dans laquelle l'électroréduction de la 3-hydroxybutanone est effectuée est compris entre 3-4.

2. Le procédé selon la revendication 1, dans lequel l'anode est une anode dimensionnellement stable.

3. Le procédé selon l'une quelconque des revendications 1-2, dans lequel l'anode est choisie dans le groupe comprenant l'oxyde d'iridium supporté sur titane, le platine supporté sur titan (Pt/Ti), et le PbO₂/Ti.

4. Le procédé selon l'une quelconque des revendications 1-3, dans lequel les métaux des groupes I B, II B et VIII B du tableau périodique sont choisis dans le groupe constitué de Fe, Co, Ni, Cu, Zn, Ru, Rh, Pd, Ag, Cd, Os, Ir, Pt, et des mélanges de ceux-ci.

5. Le procédé selon l'une quelconque des revendications 1-4, dans lequel les métaux des groupes I B, II B et VIII B du tableau périodique sont choisis dans le groupe constitué de Ni, Pd, Pt, Ru, Rh et Ir, et des mélanges de ceux-ci.

6. Le procédé selon l'une quelconque des revendications 1-5, dans lequel le matériau électrocatalytique cathodique est choisi parmi des alliages de nickel.

7. Le procédé selon l'une quelconque des revendications 1-6, dans lequel la cathode comprend de la mousse de Ni ou un métal choisi dans le groupe constitué de Ni, Pd, Pt, Ru, Rh et Ir, et des mélanges de ceux-ci déposés sur une couche de diffusion de gaz du type papier carbone ou tissu de carbone.

8. Le procédé selon la revendication 7, dans lequel la cathode est formée par revêtement du matériau électrocatalytique cathodique sur le support électriquement conducteur poreux et dans lequel la densité du métal ou de la surface d'oxyde de métal après le revêtement du support électriquement conducteur poreux est comprise entre 10 µg/cm² et 1.500 µg/cm².

9. Le procédé selon l'une quelconque des revendications 1-8, dans lequel l'électroréduction est effectuée à une densité de courant comprise entre 100 et 10.000 A/m².

10. Le procédé selon l'une quelconque des revendications 1-9, dans lequel l'électroréduction est effectuée à la pression ambiante et à une température entre la température de chambre et une température 10 °C au-dessous du point d'ébullition du milieu aqueux.

11. Le procédé selon l'une quelconque des revendications 1-10, dans lequel le milieu aqueux est de l'eau ou un mélange d'eau avec un solvant complètement ou partiellement miscible à l'eau, dans lequel la concentration d'eau dans le milieu aqueux est comprise entre 50 et 100 % en poids, et dans lequel le solvant complètement ou partiellement miscible à l'eau est choisi dans le groupe constitué des alcools, les éthers et les nitriles.

12. Le procédé selon l'une quelconque des revendications 1-11, dans lequel la concentration de l'électrolyte de support dans la solution dans laquelle l'électroréduction de la 3-hydroxybutanone est effectuée va de 0,1 à 20 % en poids, sur la base de la masse totale de la solution.

13. Le procédé selon l'une quelconque des revendications 1-12, dans lequel l'électrolyte de support dans la solution dans laquelle l'électroréduction de la 3-hydroxybutanone est effectuée est choisi dans le groupe de l'ammonium et des sels de métaux alcalins ou alcalino-terreux d'acides inorganiques.

14. Le procédé selon l'une quelconque des revendications 1-13, dans lequel la concentration de 3-hydroxybutanone dans la solution à être électrolysée est d'au moins 10 g/L, sur la base du volume total de solution à être électrolysée.

15. Le procédé selon l'une quelconque des revendications 1-14, dans lequel la quantité d'électricité en circulation pour l'électroréduction de la 3-hydroxybutanone à butane-2,3-diol est comprise entre 50 % et 150 % de la quantité théorique pour l'obtention d'une conversion de 100 % de la 3-hydroxybutanone en assumant un rendement en courant de 100 %.
